(19) Europäisches Patentamt / European Patent Office / Office européen des brevets

(11) **EP 4 215 113 A1**

(12) **EUROPEAN PATENT APPLICATION**

(43) Date of publication:
26.07.2023 Bulletin 2023/30

(21) Application number: 23152070.1

(22) Date of filing: 17.01.2023

(51) International Patent Classification (IPC):
*A61B 5/12* *(2006.01)*    *A61B 5/00* *(2006.01)*

(52) Cooperative Patent Classification (CPC):
**A61B 5/125; A61B 5/6817;** A61B 2560/0228

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **25.01.2022 EP 22153151**

(71) Applicant: **Interacoustics A/S**
**5500 Middelfart (DK)**

(72) Inventor: **NØRGAARD, Kren Monrad**
**DK-5500 Middelfart (DK)**

(74) Representative: **Demant**
**Demant A/S**
**Kongebakken 9**
**2765 Smørum (DK)**

(54)    **HIGH-FREQUENCY EAR PROBE WITH A HOLLOW TIP**

(57)    An instrument (16) configured to test the middle-ear function of a test subject, such as in tympanometry and impedance audiometry is provided. The instrument comprises an ear probe (1) for insertion in an ear of test subject, the ear probe (1) comprises an acoustic output unit comprising a receiver (2), the acoustic output unit being configured to provide a stimulus into the ear of the test subject via said receiver (2), an acoustic input unit comprising a microphone (3), the acoustic input unit being configured to receive a reflected part of said stimulus via said microphone (3) and provide an electrical input signal, an ear-probe body (4) for accommodating said microphone (3) and said receiver (2), and an ear-probe tip (10) comprising a tip opening (5) for outputting said stimulus and receiving said reflected part of the stimulus, wherein the ear-probe tip (10) comprises a sound tube (6) with a longitudinal axis (A), where said sound tube (6) provides access between a receiver opening (7) and a microphone opening (8), respectively, and said tip opening (5), which receiver opening (7) and microphone opening (8) are arranged at a distance (L) from said tip opening (5) along said longitudinal axis (A), and wherein the instrument is configured to provide said stimulus comprising one or more frequencies above 226 Hz into the ear of the test subject via said receiver (2).

FIG. 3

EP 4 215 113 A1

## Description

SUMMARY

The present application relates to an instrument configured to test the middle-ear function of a test subject.
The present application further relates to an ear probe for insertion in an ear of a test subject and for use with an instrument configured to test the middle-ear function of a test subject.

An Instrument

Tympanometry and impedance audiometry are frequently used in clinics to assess the middle-ear function of a test subject (a patient). Such measurements are usually conducted using an ear probe consisting of at least a loudspeaker (i.e., a receiver) and a microphone.
These internal ear-probe transducers are connected to the ear canal through a detachable ear-probe tip, usually with an outer diameter of 3-4 mm, and a pump and a pressure sensor to control and alter the static pressure in the ear canal. The ear probe is inserted into the ear canal and held in place using an ear tip, usually made from a silicone/rubber material and mounted onto the ear-probe tip.
The ear tip also provides a barometric seal to the ear-canal walls which allows for pressurization of the ear canal between the ear probe and the tympanic membrane (eardrum). Because ear canals are not always cleaned beforehand, it is common that some ear wax enters the ear-probe tip during measurements. To save time between consultations in the clinic, cleanability is an essential feature of ear probes used for tympanometry and impedance audiometry.
Based on a preliminary calibration of the ear probe, standardized tympanometry measures the acoustic ear-canal volume or admittance at a single probe-tone frequency of 226 Hz [1]. In some older instruments, the ear-probe tip is simply a hollow sound tube, leading from the transducers to the ear canal, whereby ear wax can easily be removed, either from the front or from the back by detaching the ear-probe tip. This means that the sound pressure recorded by the microphone is determined by the acoustic impedance seen from the location where the acoustic paths leading from the speaker to the microphone merge inside the ear-probe tip.
Because the sound wavelength at 226 Hz is long (1.5 m) relative to typical ear-probe and ear-canal dimensions (<4 cm), the sound fields inside calibration cavities and ear canals are often nearly uniform at that frequency. Consequently, without further ado, the excess volume constituted by the hollow ear-probe tip is implicitly accounted for in the calibration procedure, and subsequent volumetric measurements are accurate.
Recent trends in tympanometry and impedance audiometry are turning more toward higher frequencies. Clinical evidence suggests that measurements of the ear-canal reflectance, i.e., the proportion of sound (the stimulus) reflected by the middle ear as a function of frequency, and derived quantities such as power absorbance may aid in diagnosing and distinguishing different middle-ear pathologies.
Measurements at higher frequencies require that the internal ear-probe transducers are connected to the ear canal through individual narrow sound-delivery tubes in the ear-probe tip, because the sound wavelength at those frequencies is shorter and the direct relation between acoustic admittance/impedance and physical volume is no longer valid.
In this way, the acoustic quantities (i.e., middle-ear-function parameters) are measured from the location where the sound enters the ear canal from the ear probe rather than inside the ear-probe tip. The small dimensions of the ear-probe-tip sound-delivery tubes mean that cleanability may be severely compromised. This seems to be a current partial limiting factor for a more widespread clinical adaptation of these more advanced diagnostic measurement methods.
Consequently, methods and devices are needed that allow for an easily cleanable ear-probe tip while still allowing measurements toward higher frequencies.
In an aspect of the present application, an instrument configured to test the middle-ear function of a test subject is provided.
For example, the middle-ear function may be tested during tympanometry and impedance audiometry.
The instrument may comprise an ear probe.
The ear probe may be suitable for insertion into an ear of a test subject (e.g., of a human, or alternatively of an animal, if required).
The ear probe may be suitable for use with an instrument configured to test the middle-ear function of a test subject.
The ear probe may comprise an acoustic output unit.
The acoustic output unit may comprise an output transducer.
The output transducer may comprise or constitute a receiver (also termed a speaker/loudspeaker).
The acoustic output unit may be configured to provide a stimulus (a sound) into the ear of the test subject via said receiver.
The acoustic output unit may be configured to provide a stimulus perceived by the test subject as an acoustic signal.
The acoustic output unit may comprise a wireless receiver for receiving a wireless signal comprising or representing said stimulus (sound signal). The wireless receiver may, e.g., be configured to receive an electromagnetic signal in the radio frequency range (3 kHz to 300 GHz). The wireless receiver may, e.g., be configured to receive an electromagnetic signal in a frequency range of light (e.g., infrared light 300 GHz to 430 THz, or visible light, e.g., 430 THz to 770 THz).
The ear probe may comprise an acoustic input unit.

The acoustic input unit may comprise an input transducer.

The input transducer may comprise or constitute a microphone.

The acoustic input unit may be configured to receive a reflected part of said stimulus.

The acoustic input unit may be configured to receive a reflected part of said stimulus via said microphone.

The acoustic input unit may be configured to provide an electrical input signal.

The acoustic input unit may be configured to provide an electrical input signal corresponding to and/or resulting from said reflected part of said stimulus.

The acoustic input unit may comprise a wireless transmitter for transmitting a wireless signal comprising or representing said reflected part of said stimulus (sound signal). The wireless transmitter may, e.g., be configured to receive an electromagnetic signal in the radio frequency range (3 kHz to 300 GHz). The wireless transmitter may, e.g., be configured to transmit an electromagnetic signal in a frequency range of light (e.g., infrared light 300 GHz to 430 THz, or visible light, e.g., 430 THz to 770 THz).

The ear probe may comprise an ear-probe body.

The ear-probe body may be suitable for (configured to) accommodating at least part of said acoustic output unit and/or of said acoustic input unit.

Accommodating may refer to (at least partly) surrounding and/or containing and/or be connected to and/or fixate at least part of said acoustic output unit and/or of said acoustic input unit.

The ear-probe body may be suitable for accommodating said microphone and said receiver.

The ear probe may comprise an ear-probe tip.

The ear-probe tip may be releasably attached/connected/engaged to/with the ear-probe body. The ear-probe tip may comprise a tip opening.

The tip opening may be configured to output said stimulus.

The tip opening may be configured to receive said reflected part of said stimulus.

The tip opening may be arranged at a distal end of the ear-probe tip. In other words, the ear-probe tip may be arranged (located) at a distance from the ear-probe body.

A proximal end of the ear-probe tip may be releasably attached/connected/engaged to/with the ear-probe body. In other words, the proximal end may be located closer than said distal end to the ear-probe body. For example, the ear-probe body may surround at least part of the proximal end of the ear-probe tip.

The ear-probe tip may comprise a sound tube.

The sound tube may have a longitudinal axis (A).

The sound tube may be a hollow tube. In other words, the sound tube may comprise an inner opening extending at least part of the length of the sound tube. Said inner opening may be open at least towards the tip opening (distal end) of the ear-probe tip.

The sound tube may provide access (sound access) between a receiver opening and said tip opening.

Receiver opening may refer to an opening/output from the receiver of the acoustic output unit, e.g., via a sound (delivery) tube.

The sound tube may provide access (sound access) between a microphone opening and said tip opening.

Microphone opening may refer to an opening/input to the microphone of the acoustic input unit, e.g., via a sound (delivery) tube.

The sound tube may surround the receiver opening and/or the microphone opening.

The receiver opening may be arranged at a distance from said tip opening along said longitudinal axis of the sound tube.

The microphone opening may be arranged at a distance from said tip opening along said longitudinal axis of the sound tube.

Said distance between the receiver opening and the microphone opening, respectively, and said tip opening (i.e., the length of the hollow part of the ear-probe tip) may determine the maximum frequency where the compensation methods (see below) yield accurate results. Larger distances (i.e., longer portions of the ear-probe tip being hollow) increases cleanability of the ear probe but decreases the maximum frequency to which the instrument is useful, and vice versa.

The instrument may be configured to provide said stimulus comprising one or more frequencies above 226 Hz into the ear of the test subject via said receiver.

Thereby, an easily cleanable ear probe, which may be operated at high frequencies, is provided.

The instrument may be configured to provide said stimulus comprising one or more frequencies up to at least 4 kHz.

The instrument may be configured to provide said stimulus comprising one or more frequencies above 750 Hz into the ear of the test subject via said receiver.

The instrument may be configured to provide said stimulus comprising one or more frequencies above 1 kHz into the ear of the test subject via said receiver.

The instrument may be configured to provide said stimulus comprising frequencies above and below 1 kHz into the ear of the test subject via said receiver.

The instrument may be configured to determine one or more middle-ear-function parameters of the ear canal of the test subject based on one or more middle-ear-function parameters measured and/or calculated at said receiver opening and at said microphone opening.

One or more middle-ear-function parameters of the ear canal of the test subject may refer to middle-ear-function parameter as would be measured at the tip opening of an ear probe with individual sound-delivery tubes for the speaker and microphone (in the ear probe tip).

The determination of one or more middle-ear-function parameters of the ear canal of the test subject may at least partly be based on acoustic Thévenin-equivalent (or alternative similar) source parameters of the ear probe.

The acoustic Thévenin-equivalent source parameters of the ear probe may be determined from a calibration procedure of the ear probe.

The determination of one or more middle-ear-function parameters of the ear canal of the test subject may at least partly be based on Norton-equivalent source parameters of the ear probe. The determination of one or more middle-ear-function parameters of the ear canal of the test subject may at least partly be based on reflectance source parameters of the ear probe.

One possible means of calibrating an ear probe to measure acoustic impedance in the ear canal involves determining its acoustic Thévenin-equivalent source parameters.

The acoustic Thévenin-equivalent source parameters may be the source pressure $P_s$ and the source impedance $Z_s$ [2].

Said source parameters may conventionally be determined based on the measured sound pressure $P_i$ in each $i$ of $M > 2$ hard-walled tubes or cavities, and analytical models of their input impedances $Z_i$ using the least-squares solution (equation 1):

$$\begin{bmatrix} P_s \\ Z_s \end{bmatrix} = \begin{bmatrix} P_1 & -Z_1 \\ \vdots & \vdots \\ P_M & -Z_M \end{bmatrix}^+ \begin{bmatrix} P_1 Z_1 \\ \vdots \\ P_M Z_M \end{bmatrix}$$

where the plus superscript denotes the pseudo-inverse matrix.

The one or more middle-ear-function-parameters measured and/or calculated at said receiver opening and microphone opening may comprise a measured sound pressure.

The one or more middle-ear-function parameters measured and/or calculated at said receiver opening and microphone opening may comprise a calculated impedance. The impedance may be calculated based on said measured sound pressure.

The one or more middle-ear-function-parameters measured and/or calculated at said receiver opening and microphone opening may comprise a calculated acoustic admittance.

The one or more middle-ear-function-parameters measured and/or calculated at said receiver opening and microphone opening may comprise a calculated reflectance.

The one or more middle-ear-function-parameters of the ear canal of the test subject may be based on measured voltage at the microphone terminals of the microphone.

For example, said one or more middle-ear-function-parameters may be based on measured voltage at the microphone terminals when the outcome of a measurement is not an absolute quantity, i.e., sound pressure. Acoustic impedance, reflectance, etc. are relative quantities, i.e., they consist of ratios of two absolute quantities, so the microphone sensitivity cancels out. In this way, a calibration may be conducted based on voltages on the microphone [3].

The determination of one or more middle-ear-function parameters of the ear canal of the test subject may comprise propagating a said calculated impedance at said receiver opening and microphone opening using a transmission line.

An acoustic transmission line is one example of a type of two-port network that relates the sound pressure $P_{\text{in}}$ and volume flow $U_{\text{in}}$ at the input terminals to those at the output terminals $P_{\text{out}}$ and $U_{\text{out}}$ by a 2-by-2 transfer matrix A with elements $a_{ij}$, (equation 2):

$$\begin{bmatrix} P_{\text{in}} \\ U_{\text{in}} \end{bmatrix} = \begin{bmatrix} a_{11} & a_{12} \\ a_{21} & a_{22} \end{bmatrix} \begin{bmatrix} P_{\text{out}} \\ -U_{\text{out}} \end{bmatrix}.$$

Alternative examples of types of two-port networks may be based on scattering parameters, i.e., incident and reflected waves that can be used for calculations based on reflectance.

The exemplary formulation of equation 2 is preferable because transmission-lines segments can easily be cascaded by multiplying their transfer matrices. Analytical expressions exist for some types of acoustic transmission lines, e.g., a uniform tube, (equation 3):

$$\mathbf{A} = \begin{bmatrix} \cosh \gamma l & Z_0 \sinh \gamma l \\ 1/Z_0 \sinh \gamma l & \cosh \gamma l \end{bmatrix},$$

where $\gamma$ is the propagation constant, $l$ is the length, and $Z_0$ is the characteristic impedance of the tube.

Alternative two-port networks may describe acoustic lumped elements (i.e., acoustic compliances and masses), or non-uniform transmission lines.

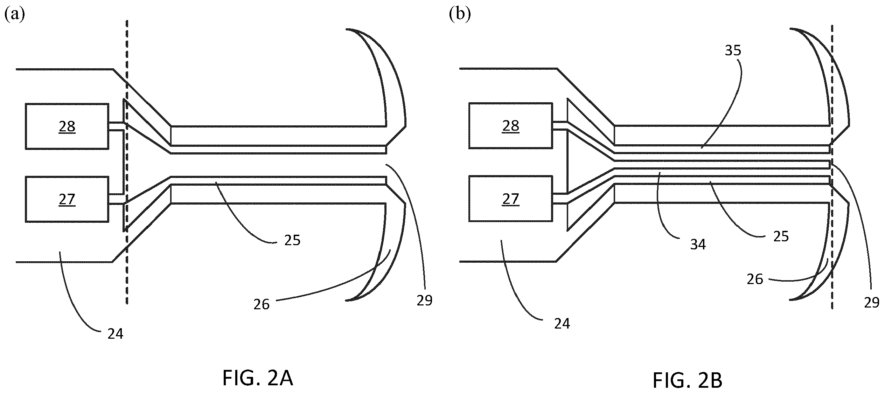

When the ear-probe tip is hollow, measured acoustic quantities are determined by the acoustic impedance as seen from the location where the microphone and receiver tubes merge (as, e.g., illustrated as the dotted line in FIG. 2A). For an ear-probe tip with individual sound tubes, the measured pressure corresponds to that due to the input impedance of each calibration cavity $Z_i$. However, for a hollow sound tube, the pressure corresponds to that of the calibration cavity as seen through the probe tip $Z_i^{\text{tip}}$. Having determined the acoustic transmission-line matrix for a given ear-probe tube A with elements $a_{11}$, $a_{12}$, $a_{21}$, and $a_{22}$, the load impedance to be used for calibrating the ear probe can be calculated by terminating the ear-probe-tip transmission line by the analytical cavity impedance $Z_i$ (equation 4):

$$Z_i^{\text{tip}} = \frac{a_{11}Z_i + a_{12}}{a_{21}Z_i + a_{22}}.$$

Accordingly, the obtained source parameters $P_s$ and $Z_s$ represent those as seen from inside the ear-probe tip.

When the ear probe is inserted into the ear canal after a successful calibration, the sound pressure $P_{\text{ec}}^{\text{tip}}$ is measured and the impedance $Z_{\text{ec}}^{\text{tip}}$ can be calculated based on the obtained source parameters using the conventional expression (equation 5):

$$Z_{\text{ec}}^{\text{tip}} = Z_s \frac{P_{\text{ec}}^{\text{tip}}}{P_s - P_{\text{ec}}^{\text{tip}}}.$$

Note again that both $P_{\text{ec}}^{\text{tip}}$ and $Z_{\text{ec}}^{\text{tip}}$ represent those where the signals merge (i.e., the receiver opening and the microphone opening merge) inside the ear-probe tip and not those in the ear canal. Next, the (complex) ear-canal impedance $Z_{\text{ec}}$ can be calculated by propagating the ear-probe tip transmission line out of the impedance $Z_{\text{ec}}^{\text{tip}}$ (equation 6):

$$Z_{\text{ec}} = \frac{a_{12} - a_{22}Z_{\text{ec}}^{\text{tip}}}{a_{21}Z_{\text{ec}}^{\text{tip}} - a_{11}}.$$

The middle-ear-function parameters of the ear canal of the test subject may comprise an ear-canal impedance ($Z_{\text{ec}}$).

The middle-ear-function parameters of the ear canal of the test subject may comprise a complex acoustic ear-canal volume ($V_{\text{ec}}$).

For example, a complex acoustic ear-canal volume ($V_{\text{ec}}$) may be defined by (equation 7):

$$V_{\text{ec}} = \frac{\rho c^2}{j\omega Z_{\text{ec}}},$$

The middle-ear-function parameters of the ear canal of the test subject may comprise an ear-canal power absorbance ($A_{\text{ec}}$).

For example, an ear canal power absorbance ($A_{\text{ec}}$) may be defined by (equation 8):

$$A_{\text{ec}} = 1 - |R_{\text{ec}}|^2,$$

The middle-ear-function parameters of the ear canal of the test subject may comprise an (complex) ear-canal reflectance ($R_{\text{ec}}$).

For example, an ear-canal reflectance ($R_{\text{ec}}$)

may be defined by (equation 9):

$$R_{\text{ec}} = \frac{Z_{\text{ec}} - Z_0}{Z_{\text{ec}} + Z_0},$$

The middle-ear-function parameters of the ear canal of the test subject may comprise an (complex) ear-canal admittance ($Y_{\text{ec}}$).

For example, an ear-canal admittance ($Y_{\text{ec}}$) may be related to the ear-canal impedance ($Z_{\text{ec}}$) by (equation 10):

$$Y_{\text{ec}} = \frac{1}{Z_{\text{ec}}}.$$

The middle-ear-function parameters of the ear canal of the test subject may comprise a combination of one or more of either the ear-canal impedance ($Z_{\text{ec}}$), the complex ear-canal volume ($V_{\text{ec}}$), the ear-canal power absorbance ($A_{\text{ec}}$), the ear-canal admittance ($Y_{\text{ec}}$), and/or the ear-canal reflectance ($R_{\text{ec}}$).

The receiver opening and the microphone opening may be arranged at equal distance from said tip opening in a direction along the longitudinal axis of the sound tube.

Equal distance may refer to that the distance/length from the receiver opening to the tip opening is similar to the distance/length from the microphone opening to the tip opening. The receiver opening and the microphone opening may merge at a point inside the sound tube at a distance/length from the tip opening.

The diameter/cross-sectional area of the receiver opening and/or of the microphone opening may be less than the diameter/cross-sectional area of the ear-probe tip and/or of the sound tube.

Thereby, the sound tube may surround/enclose the receiver opening and microphone opening so that the receiver opening and the microphone opening are not directly exposed to the surroundings of the ear probe, which could otherwise potentially result in an at least partly clogging of the receiver opening and microphone opening.

The instrument may be configured to provide the stimulus as a wideband click.

For example, the wideband click may be applied in the frequency range from 0.125 kHz to 4 kHz.

The instrument may further comprise an ear tip.

The ear tip may be suitable for releasable attachment to the ear-probe tip (and/or the ear-probe body).

Releasable attachment may refer to that the ear tip may easily be attached to/connected to/engage with the ear-probe tip (and/or the ear-probe body) and easily be detached/released again after use.

The attachment/connection/engagement may be based on frictional force between the ear tip and the ear-probe tip (and/or the ear-probe body). For example, the ear tip may have to be forced over an outer surface of the ear-probe tip (and/or the ear-probe body), e.g., over an outer surface of the sound tube. For example, at least part of the ear tip may have to be forced into a recess and/or over a protrusion of the ear-probe tip (and/or the ear-probe body).

The engagement/connection may, e.g., be based on a recess of the ear tip engaging with a protrusion of ear-probe tip (and/or the ear-probe body), or vice versa. For example, the ear-probe tip (and/or the ear-probe body) may comprise a thread onto which the ear tip may be screwed (e.g., via female thread).

The ear tip may be configured to provide a barometric seal toward the ear-canal walls in the ear of the test subject.

For example, the ear tip may be made from a silicone/rubber material. Thereby, the ear tip may be elastic which allows for that the ear tip may shape itself according to the part of the ear-canal walls it contacts to after insertion into the ear. Hereby, the ear tip may provide a barometric seal to the ear-canal walls, which allows for pressurization of the ear.

The ear-probe tip may be releasably connected to the ear-probe body.

Releasably connected may (as for releasable attachment above) refer to that the ear-probe tip may easily be attached to/connected to/engage with the ear-probe body and easily be detached/released/decoupled again.

The connection/attachment/engagement may be based on frictional force between the ear-probe tip and the ear-probe body. For example, the ear-probe tip may have to be forced over an outer surface of the ear-probe body, or vice versa. For example, at least part of the ear-probe tip may have to be forced into a recess and/or over a protrusion of the ear-probe body. The connection/attachment/engagement may, e.g., be based on a recess of the ear-probe tip engaging with a protrusion of ear-probe body, or vice versa. For example, the ear-probe body may comprise a thread onto which the ear-probe tip may be screwed (e.g., via female thread). Thereby, part of the internal tubing (e.g., comprising the receiver opening and the microphone opening) may be attached to the ear-probe body, and may form a closed tubing when the ear-probe tip is attached to the ear-probe body. In this way, when the ear-probe tip (and therefore also the sound tube) is detached/decoupled/released, it may be completely hollow and can easily be cleaned.

Furthermore, if ear wax has entered the ear-probe tip, and, e.g., the receiver opening and/or the microphone opening (and the tubing leading to the microphone and the receiver), attached to the ear-probe body, the receiver opening and the microphone opening can easily be cleaned when the ear-probe tip is detached because the ear-probe tip may form the outer wall of the receiver opening and/or the microphone opening (and the tubing leading to the microphone and the receiver) which is now open.

The instrument may further comprise a pump. The pump may be configured to provide a pressure higher than and/or lower than ambient pressure (e.g., approx. 1 bar), and/or configured to maintain ambient pressure in the ear, when the ear probe has been inserted into the ear. The pump may be configured to maintain a static pressure (higher, lower, or equal to ambient pressure) for at least a pre-set time interval in the ear. The pump may be configured to vary the pressure in the ear according to a pre-set schedule.

The instrument may further comprise a pressure sensor.

The pressure sensor may be configured to measure the pressure in the ear of a test subject when the ear probe has been inserted into the ear.

The instrument may be configured to control the pressure in the ear canal of the test subject by the pump and the pressure sensor.

The instrument may further comprise a control unit. The control unit may be configured to control the pump based on the pressure measured by the pressure sensor. The control unit may be configured to control the pump according to a pre-determined schedule and based on the pressure measured in the ear of the test subject by the pressure sensor.

The pump and the pressure sensor may be in fluid communication with the ear-probe tip. For example, the pressure sensor may be connected to a tube connecting the pump to the ear canal using a T-connector and an additional piece of tube.

The instrument may further comprise one or more wax guards.

The one or more wax guards may be arranged at said receiver opening, and/or in a tubing leading from the receiver to the receiver opening.

The one or more wax guards may be arranged at said microphone opening, and/or in a tubing leading from the microphone to the microphone opening.

The one or more wax guards may be arranged in the sound tube at any location between the receiver and microphone opening and the tip opening.

Thereby, ear wax and/or other debris is prevented from entering and damaging the transducers (microphone and receiver).

The instrument may further comprise one or more wax guards arranged at the tip opening. Thereby, ear wax and/or other debris is prevented entirely from entering the ear-probe tip, and accordingly also from entering and damaging the transducers.

The instrument may further comprise a signal generator for providing said stimulus.

The signal generator may be connected to the acoustic output unit and to the receiver either via a wired connection or wirelessly (e.g., based on Bluetooth technology (e.g., Bluetooth Low-Energy technology), or Ultra WideBand (UWB) technology). The signal generator may provide said stimulus to the ear of the test subject via the receiver.

The instrument may further comprise a processor configured to carry out the determination of said one or more middle-ear-function parameters of the ear canal of the test subject.

The processor may be connected to the acoustic input unit and to the microphone either via a wired connection or wirelessly. The processor may carry out said determination based at least partly on the electric input signal provided by the acoustic input unit generated/determined from the reflected part of said stimulus.

In an aspect of the present application, an ear probe is provided.

The ear probe may be suitable for insertion in an ear of a test subject and for use with an instrument configured to test the middle-ear function of a test subject according to the description above.

The ear probe may comprise an acoustic output unit comprising a receiver, the acoustic output unit being configured to provide a stimulus into the ear of the test subject via said receiver. The ear probe may comprise an acoustic input unit comprising a microphone, the acoustic input unit being configured to receive a reflected part of said stimulus via said microphone and provide an electrical input signal.

The ear probe may comprise an ear-probe body for accommodating said microphone and said receiver.

The ear probe may comprise a tip opening for outputting said stimulus and receiving said reflected part of the stimulus.

The ear-probe tip may comprise a sound tube with a longitudinal axis, where said sound tube provides access between a receiver opening and a microphone opening, respectively, and said tip opening, which receiver opening and microphone opening are arranged at a distance from said tip opening along said longitudinal axis.

The ear probe may be configured to provide said stimulus at frequencies above 226 Hz into the ear of the test subject via said receiver.

Use:

In an aspect, use of an instrument and ear probe as described above, in the 'detailed description of embodiments' and in the claims, is moreover provided. Use may be provided in an instrument comprising one or more ear probes.

A method:

In an aspect, a method to test the middle-ear function of a test subject, such as in tympanometry and impedance audiometry, is provided.

The method may comprise an instrument as described above comprising an ear probe as described above for insertion in an ear of a test subject.

The method may comprise providing a stimulus into the ear of the test subject by an acoustic output unit comprising a receiver.

The method may comprise receiving a reflected part of said stimulus by an acoustic input unit comprising a microphone.

The method may comprise providing an electrical input signal by the acoustic input unit.

The method may comprise providing an ear-probe body for accommodating said microphone and said receiver.

The method may comprise providing an ear-probe tip comprising a tip opening for outputting said stimulus and receiving said reflected part of the stimulus.

The ear-probe tip may comprise a sound tube with a longitudinal axis, where said sound tube provides access between a receiver opening and a microphone opening, respectively, and said tip opening.

The receiver opening and microphone opening may be arranged at a distance from said tip opening along said longitudinal axis.

The method may comprise providing said stimulus comprising one or more frequencies above 226 Hz into the ear of the test subject via said receiver.

It is intended that some or all of the structural features of the instrument and ear probe described above, in the 'detailed description of embodiments' or in the claims can be combined with embodiments of the method, when appropriately substituted by a corresponding process and vice versa. Embodiments of the method have the same advantages as the corresponding instrument and ear probe.

A computer-readable medium or data carrier:

In an aspect, a tangible computer-readable medium (a data carrier) storing a computer program comprising program-code means (instructions) for causing a data-processing system (a computer) to perform (carry out) at least some (such as a majority or all) of the (steps of the) method described above, in the 'detailed description of embodiments' and in the claims, when said computer program is executed on the data processing system is furthermore provided by the present application.

A computer program:

A computer program (product) comprising instructions which, when the program is executed by a computer, cause the computer to carry out (steps of) the method described above, in the 'detailed description of embodiments' and in the claims is furthermore provided by the present application.

A data processing system:

In an aspect, a data processing system comprising a processor and program code means for causing the processor to perform at least some (such as a majority or all) of the steps of the method described above, in the 'detailed description of embodiments' and in the claims is furthermore provided by the present application.

An APP:

In a further aspect, a non-transitory application, termed an APP, is furthermore provided by the present disclosure. The APP comprises executable instructions configured to be executed on an auxiliary device to implement a user interface for an instrument described above in the 'detailed description of embodiments', and in the claims. The APP may be configured to run on cellular phone, e.g., a smartphone, or on another portable device allowing communication with said instrument.

BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:

FIG. 1 shows an exemplary instrument according to the present application.

FIGS. 2A and 2B show cross sections of exemplary ear probes.

FIG. 3 shows a cross-section of an exemplary ear probe according to the present application.

FIG. 4 shows exemplary measurements of complex ear-canal volume $V_{ec}$ and absorbance in an occluded-ear simulator using a hollow ear-probe tip, with and without compensation, and a standard ear-probe tip with individual tubing.

The figures are schematic and simplified for clarity, and they just show details which are essential to the understanding of the disclosure, while other details are left out. Throughout, the same reference signs are used for identical or corresponding parts.

Further scope of applicability of the present disclosure will become apparent from the detailed description given hereinafter. However, it should be understood that the detailed description and specific examples, while indicating preferred embodiments of the disclosure, are given by way of illustration only. Other embodiments may become apparent to those skilled in the art from the following detailed description.

DETAILED DESCRIPTION OF EMBODIMENTS

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the instrument and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

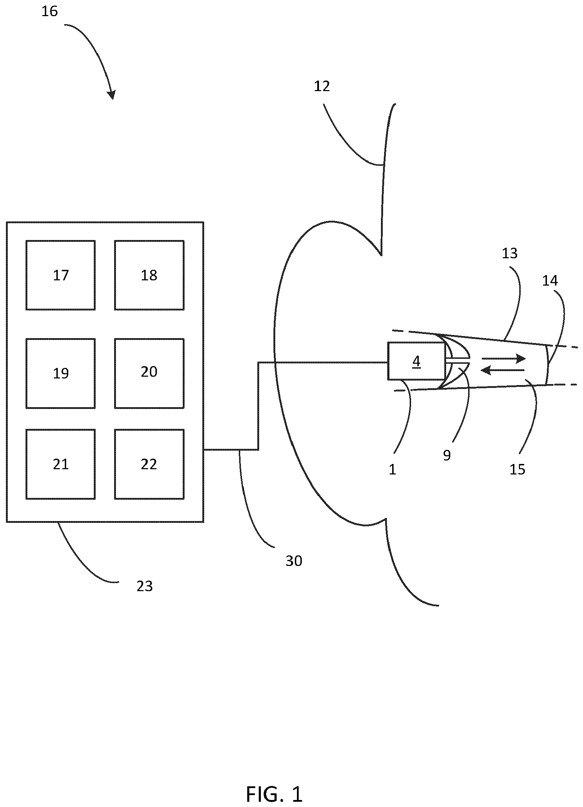

FIG. 1 shows an exemplary instrument according to the present application.

In FIG. 1, the instrument 16 is shown to be located at and in connection with the head 12 of a test subject. A test subject may refer to a human, but animals may also be considered.

The instrument 16 may be configured to test the middle-ear function of the test subject, such as in tympanometry and impedance audiometry.

The instrument 16 is shown to comprise two parts, but may of course be combined to only one part, or alternatively be divided into several parts.

In FIG. 1, a first part may be arranged inside the ear canal 15 of the test subject. Alternatively, or additionally, the first part may be arranged at least partly outside the ear canal 15. The first part is shown to be an ear probe 1 suitable for insertion in the ear.

The ear probe 1 may comprise an ear tip 9, which may be releasably attached to an ear-probe body 4. When the ear probe 1 has been inserted into the ear canal 15, the ear tip 9 may provide a barometric seal toward the ear-canal walls 13 of the ear canal 15. The ear probe 1 may provide a stimulus into the ear of the test subject in a direction towards the eardrum 14 and receive a reflected part of said stimulus, as indicated by the two arrows located in the ear canal 15.

A second part 23 of the instrument is shown to be arranged outside the ear canal 15 of the test subject. Alternatively, or additionally, the second part 23 (or at least some elements of the second part 23) could be combined with the ear probe 1.

The second part 23 may comprise a pump 17 and a pressure sensor 18 by which the instrument 1 can control the pressure in the ear canal 15 in the space between the ear probe 1 and the eardrum 14. Therefore, the pump 17 and a pressure sensor 18 may be in fluid communication with the ear canal 15. The pump 17 and a pressure sensor 18 may provide a pressure equal to, above, or below ambient pressure.

**EP 4 215 113 A1**

The second part 23 may comprise a signal generator 19 for generating said stimulus to be introduced into the ear canal 15 of the test subject.

The second part 23 may comprise an acoustic output unit 20. The acoustic output unit 20 may be connected to the signal generator 19 and may comprise a receiver (not shown) located in the ear probe 1. The acoustic output unit 20 may be configured to introduce said stimulus which is generated by the signal generator 19 into the ear of the test subject, via said receiver.

The second part 23 may comprise a processor 21 configured to carry out a determination of one or more middle-ear-function parameters of the ear canal of the test subject.

The second part 23 may comprise an acoustic input unit 22. The acoustic input unit 22 may be connected to the processor 21 and may comprise a microphone (not shown) located in the ear probe 1. The acoustic input unit 22 may be configured to receive a reflected part of said stimulus via said microphone 3 and provide an electrical input signal to said processor 21, based on which the processor 21 may carry out said determination.

As indicated in FIG. 1, the second part 23 may be connected to the first part (the ear probe 1). The first and second part 23 may be connected by a wired or a wireless connection 30.

FIGS. 2A and 2B show cross-sections of exemplary ear probes.

Each of the two ear probes are shown to comprise an ear-probe body 24, an ear-probe tip 25, and an ear tip 26.

Inside the ear-probe body 24, a receiver 27 for providing a stimulus into the ear of a test subject and a microphone 28 for receiving a reflected part of said stimulus are arranged.

In FIG. 2A, an opening of the receiver 27 and an opening of the microphone 28 are located at equal distance from the tip opening 29 of the ear probe, as is indicated by the dotted vertical line. Thereby, the receiver provides the stimulus to (and the microphone receives the reflected part from) the hollow ear-probe tip 25 at a distance from the tip opening 29. One or more middle-ear-function parameters are then determined at the location indicated by the dotted vertical line inside the ear-probe tip 25.

In FIG. 2B, separate stimulus-delivery tubes 34,35 lead from the receiver 27 and the microphone 28, respectively, to the tip opening 29 of the ear probe (indicated by the dotted vertical line), and do not combine/merge inside the ear-probe tip 25. Thereby, the stimulus is provided directly to (and received directly from) the ear canal of the test subject. One or more middle-ear-function parameters are then determined at the location indicated by the dotted vertical line at the tip opening 29.

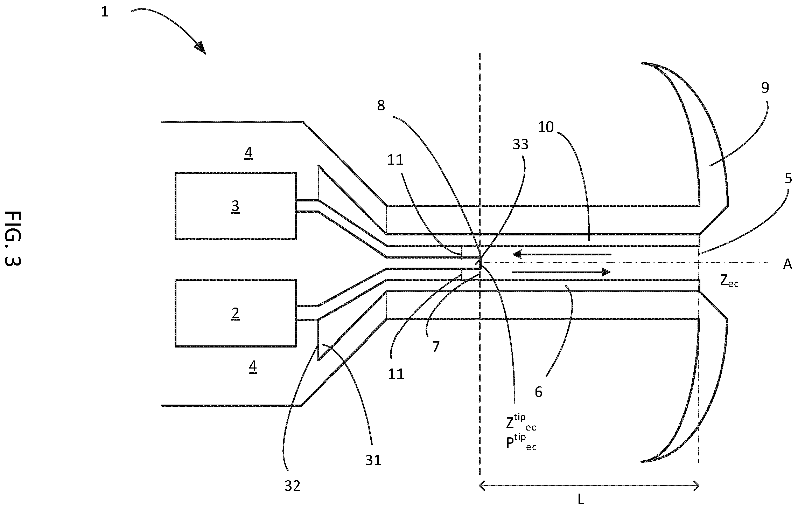

FIG. 3 shows a cross-section of an exemplary ear probe according to the present application.

In FIG. 3, the ear probe 1 comprises an ear-probe body 4, an ear-probe tip 10, and an ear tip 9.

The ear-probe body 4 accommodates a microphone 3 and a receiver 2. The receiver 2 may form part of the acoustic output unit of the instrument. The microphone 3 may form part of the acoustic input unit of the instrument.

The ear-probe tip 10 may comprise a tip opening 5 (at one (distal) end of the ear-probe tip 10). The tip opening 5 opens up to the ear canal, when the ear probe 1 is inserted in the ear canal. The tip opening 5 facilitates the output of a stimulus generated in the signal generator to the ear canal and the receiving of a reflected part of the stimulus.

At an opposite (proximal) end 31 of the ear-probe tip 10, the ear-probe tip 10 is connected to the ear-probe body 4. The ear-probe tip 10 and the ear-probe body 4 may be connected in a releasable manner, so that the user (operator) operating the instrument, e.g., a hearing-care professional, may easily decouple/detach the ear-probe tip 10, when e.g., the ear-probe tip 10 needs cleaning or replacement. In FIG. 3, the ear-probe tip 10 (i.e., said opposite end 31) is shown to protrude into a recess 32 of the ear-probe body 4.

The ear-probe tip 10 may comprise a sound tube 6 with a longitudinal axis A. The sound tube 6 may provide access between a receiver opening 7 and a microphone opening 8, respectively, and said tip opening 5. The sound tube 6 may be at least partly hollow. In FIG. 3, it is illustrated that the sound tube 6 is hollow between the receiver opening 7 and microphone opening 8 and the tip opening 5. The receiver opening 7 and the microphone opening 8 may be arranged at a distance L from said tip opening 5 seen along said longitudinal axis A.

The ear probe 1 may further comprise an ear tip 9. The ear tip 9 may be formed from a flexible material and thereby provide a barometric seal toward the ear-canal walls in the ear. The ear tip 9 may be releasably attached/connected to the ear-probe tip 10. Thereby, the ear tip 9 may be removed from the ear-probe tip 10 and be replaced by a new ear tip 9 after use.

The ear probe 1 may further comprise one or more wax guards 11 (e.g., wax filters) to prevent, e.g., cerumen from entering the ear-probe tip 10, or at least from entering the microphone 3 and receiver 2. The one or more wax guards 11 may be arranged at any location between the receiver 2 or microphone 3 and the tip opening 5. In FIG. 3, wax guards are arranged in the tubing between the receiver 2/microphone 3 and their respective openings 7,8. However, it is also contemplated that the wax guards may be arranged at the ear-probe tip 5 to completely prevent cerumen from entering the ear probe 1.

The receiver opening 7 and the microphone opening 8 are shown to be located inside the sound tube 6. The location of said receiver opening 7 and microphone opening 8 are determined by a protrusion 33 of the ear-probe body 4, which extends into said sound tube 6, when the ear-probe tip 10 is attached to the ear-probe body 4.

During testing of middle-ear function of the test subject, the sound pressure $P^{tip}_{ec}$ is measured at the location of the receiver opening 7 and the microphone opening 8 (indicated by the vertical dotted line). The corresponding impedance $Z^{tip}_{ec}$ can be calculated by the following equation (equation 5), where $P_s$ and $Z_s$ are predetermined source parameters of the ear-probe tip 10.

$$Z^{tip}_{ec} = Z_s \frac{P^{tip}_{ec}}{P_s - P^{tip}_{ec}}.$$

From this, the ear-canal impedance $Z_{ec}$ can be calculated by propagating the ear-probe tip transmission line out of the impedance $Z^{tip}_{ec}$ according to the following (equation 6):

$$Z_{ec} = \frac{a_{12} - a_{22} Z^{tip}_{ec}}{a_{21} Z^{tip}_{ec} - a_{11}}.$$

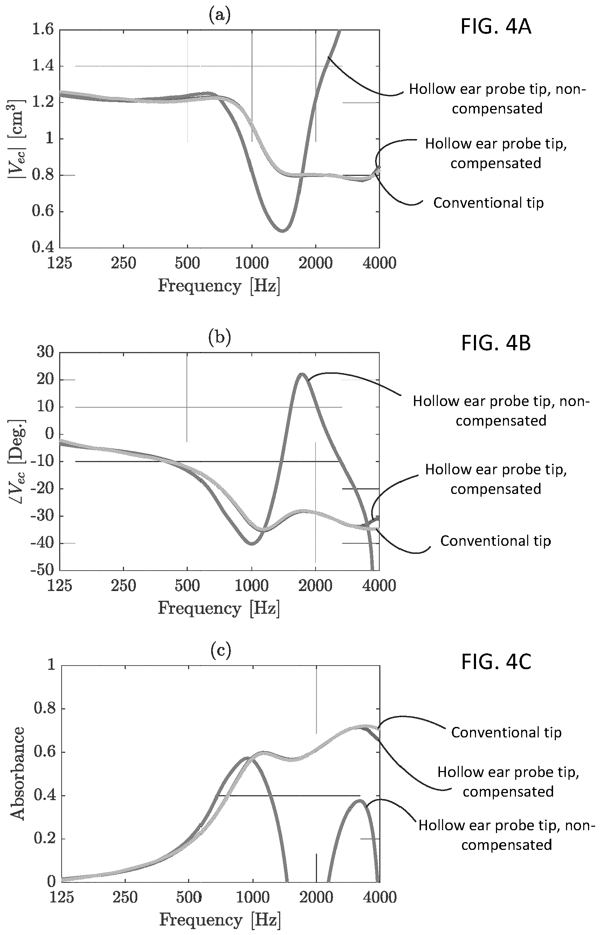

FIGS. 4A-4C show exemplary measurements of complex ear-canal volume $V_{ec}$ and absorbance in an occluded-ear simulator using a hollow ear-probe tip, with (see FIG. 3) and without compensation (see FIG. 2A), and a standard ear-probe tip (see FIG. 2B) with individual tubing.

Initial calibration of the ear probes and following measurements to test the middle-ear function using the ear probes were conducted with an ear-probe tip that had a hollow portion of 6 mm, i.e., with a hollow sound tube of a length (L) of 6 mm from the receiver opening and the microphone opening, respectively, to the tip opening.

The measurements were conducted using both the proposed compensation scheme (see equations 5 and 6 above for the determination of ear-canal impedance ($Z_{ec}$)) accounting for the hollow ear-probe tip and using the conventional methods (with an ear probe in line with FIG. 2A).

Furthermore, comparison measurements were made using a standard tip with individual tubing (with an ear probe in line with FIG. 2B) and conventional calibration methods as described in [4].

Results are shown in FIG. 4A-4C in terms of (see also above for further detail) the complex ear-canal volume:

$$V_{ec} = \frac{\rho c^2}{j \omega Z_{ec}},$$

and power absorbance:

$$A_{ec} = 1 - |R_{ec}|^2,$$

where the ear-canal reflectance:

$$R_{ec} = \frac{Z_{ec} - Z_0}{Z_{ec} + Z_0},$$

in a standardized occluded-ear simulator [5].

The results clearly demonstrate how using the hollow ear-probe tip and the proposed compensation methods are in line with those using a conventional method with the standard ear-probe tip (see FIG. 2B) up to 4 kHz. Conversely, results using the hollow ear-probe tip without compensation (see FIG. 2A) becomes increasingly unreliable above approximately 750 Hz.

It is intended that the structural features of the devices described above, either in the detailed description and/or in the claims, may be combined with steps of the method, when appropriately substituted by a corresponding process.

As used, the singular forms "a," "an," and "the" are intended to include the plural forms as well (i.e., to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element, but an intervening element may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method are not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The claims are not intended to be limited to the aspects shown herein but are to be accorded the full scope consistent with the language of the claims, wherein reference to an element in the singular is not intended to mean "one and only one" unless specifically so stated, but rather "one or more." Unless specifically stated otherwise, the term "some" refers to one or more.

REFERENCES

[1] IEC 60645-5 (2004). Instruments for the measurement of aural acoustic impedance/admittance (International Electrotechnical Commission, Geneva, Switzerland).
[2] Allen, J. B. (1986). Measurement of eardrum acoustic impedance. In J. Allen, J. Hall, A. Hubbard, S. Neely, & A. Tubis (Eds.), Peripheral Auditory Mechanisms (pp. 44-51). Springer-Verlag.
[3] Nørgaard et al. (2018). A coupler-based calibration method for ear-probe microphones. J. Acoust. Soc. Am., 144(4), 2294-2299.
[4] Nørgaard, K. R., Fernandez-Grande, E., & Laugesen, S. (2017). Incorporating evanescent modes and flow losses into reference impedances in acoustic Thévenin calibration. J. Acoust. Soc. Am., 142(5), 3013-3024.
[5] IEC 60318-4 (2010). Occluded-ear simulator for the measurement of earphones coupled to the ear by means of ear inserts (International Electrotechnical Commission, Geneva, Switzerland).

**Claims**

1. Instrument (16) configured to test the middle-ear function of a test subject, such as in tympanometry and impedance audiometry, where the instrument comprises an ear probe (1) for insertion in an ear of test subject, the ear probe (1) comprising:

    - an acoustic output unit comprising a receiver (2), the acoustic output unit being configured to provide a stimulus into the ear of the test subject via said receiver (2),
    - an acoustic input unit comprising a microphone (3), the acoustic input unit being configured receive a reflected part of said stimulus via said microphone (3) and provide an electrical input signal,
    - an ear-probe body (4) for accommodating said microphone (3) and said receiver (2), and
    - an ear-probe tip (10) comprising a tip opening (5) for outputting said stimulus and receiving said reflected part of the stimulus,
    - wherein the ear-probe tip (10) comprises a sound tube (6) with a longitudinal axis (A), where said sound tube (6) provides access between a receiver opening (7) and a microphone opening (8), respectively, and said tip opening (5), and where said receiver opening (7) and microphone opening (8) are arranged at a distance (L) from said tip opening (5) along said longitudinal axis (A), and
    - wherein the instrument is configured to provide said stimulus comprising one or more frequencies above 226 Hz into the ear of the test subject via said receiver (2).

2. Instrument (16) according to claim 1, wherein the instrument is configured to provide said stimulus comprising one or more frequencies up to at least 4 kHz.

3. Instrument (16) according to any one of the preceding claims, wherein the instrument is configured to determine one or more middle-ear-function parameters of the ear canal of the test subject based on one or more middle-ear-function parameters measured and/or calculated at said receiver opening (7) and microphone opening (8).

4. Instrument (16) according to claim 3, wherein determining one or more middle-ear-function parameters of the ear canal of the test subject is at least partly based on source parameters of the ear probe (1), as determined from a calibration procedure of the ear probe (1), such as acoustic Thévenin-equivalent source parameters, Norton-equivalent source parameters, or reflectance source parameters.

5. Instrument (16) according to any one of claims 3-4, wherein the one or more middle-ear-function parameters measured and/or calculated at said receiver opening (7) and microphone opening (8) comprises a measured sound pressure, a calculated impedance, a calculated acoustic admittance, and a calculated reflectance.

6. Instrument (16) according to any one of claims 3-5, wherein, determining one or more middle-ear-function parameters of the ear canal of the test subject, comprises propagating a transmission line from said calculated impedance at said receiver opening (7) and microphone opening (8).

7. Instrument (16) according to any one of the preceding claims, wherein the middle-ear-function parameters of the ear canal of the test subject comprises one or more of an ear-canal impedance ($Z_{ec}$), a complex ear-canal volume ($V_{ec}$), an ear-canal admittance ($Y_{ec}$), an ear-canal reflectance ($R_{ec}$), and/or an ear-canal power absorbance ($A_{ec}$).

8. Instrument (16) according to any one of the preceding claims, wherein the receiver opening (7) and the microphone opening (8) are arranged at equal distance (L) from said tip opening (5) along said longitudinal axis (A).

9. Instrument (16) according to any one of the preceding claims, wherein the instrument is configured to provide said stimulus as a wideband click.

10. Instrument (16) according to any one of the preceding claims, wherein the instrument further comprises an eartip (9) for releasable attachment to the ear-probe tip (10), where the ear tip is configured to provide a barometric seal toward the ear-canal walls in the ear of the test subject.

11. Instrument (16) according to any one of the preceding claims, wherein the ear-probe tip (10) is releasably connected to said ear-probe body (4).

12. Instrument (16) according to any one of the preceding claims, wherein the instrument further comprises a pump and a pressure sensor, where the instrument is configured to control the pressure in the ear canal of the test subject by the pump and the pressure sensor.

13. Instrument (16) according to any one of the preceding claims, wherein the instrument further comprises one or more wax guards (11) arranged at said receiver opening (7) and/or at said microphone opening (8).

14. Instrument (16) according to any one of the preceding claims, wherein the instrument further comprises one or more wax guards arranged at said tip opening (5).

15. Instrument (16) according to any one of the preceding claims, wherein the instrument further comprises a signal generator for providing said stimulus, and a processor configured to carry out the determination of said one or more middle-ear-function parameters of the ear canal of the test subject.

16. Method of testing the middle-ear function of a test subject, such as in tympanometry and impedance audiometry, the method comprising:

   - providing a stimulus at one or more frequencies above 226 Hz into the ear of the test subject by an acoustic output unit comprising a receiver,
   - receiving a reflected part of said stimulus by an acoustic input unit comprising a microphone,
   - providing an electrical input signal based on said reflected part of said stimulus by the acoustic input unit,
   - providing an ear-probe tip comprising a tip opening for outputting said stimulus and

   - receiving said reflected part of said stimulus, where the ear-probe tip comprises a sound tube with a longitudinal axis, and where said sound tube provides access between a receiver opening and a microphone opening, respectively, and said tip opening, and where the receiver opening and the microphone opening are arranged at a distance from said tip opening along said longitudinal axis.

17. Method according to claim 16, the method comprises determining an ear-canal impedance $Z_{ec}$ by propagating an ear-probe tip transmission line from an impedance calculated at said receiver opening and said microphone opening.

18. A computer program comprising instructions which, when the program is executed by a computer, cause the computer to carry out the method according to any of claims 16 or 17.

16

12

13

14

17    18

19    20

21    22

4

1    9    15

30

23

FIG. 1

(a)

28

27

24

25

26

29

FIG. 2A

(b)

35

28

27

24

34

25

26

29

FIG. 2B

FIG. 3

(a)

FIG. 4A

Hollow ear probe tip, non-compensated

Hollow ear probe tip, compensated

Conventional tip

(b)

FIG. 4B

Hollow ear probe tip, non-compensated

Hollow ear probe tip, compensated

Conventional tip

(c)

FIG. 4C

Conventional tip

Hollow ear probe tip, compensated

Hollow ear probe tip, non-compensated

Europäisches
Patentamt

European
Patent Office

Office européen
des brevets

**EUROPEAN SEARCH REPORT**

Application Number

EP 23 15 2070

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/257188 A1 (FATHER FLANAGANS BOYS HOME DOING BUSINESS AS BOYS TOWN NATIONAL RES HO) 24 December 2020 (2020-12-24) | 1-7,9, 10,12, 15-18 | INV. A61B5/12 A61B5/00 |
| Y | * page 3, lines 21-24 * <br> * page 4, lines 14-27 * <br> * page 5, lines 6-10,17-25 * <br> * page 7, lines 19-21 * <br> * page 9, lines 23-30 * <br> * page 12, line 19 - page 13, line 3 * <br> * page 14, lines 1-6 * <br> * page 23, line 27 - page 24, line 2 * <br> * figure 1 * <br> ----- | 13,14 | |
| X | US 2007/112279 A1 (ISEBERG STEVE [US] ET AL) 17 May 2007 (2007-05-17) <br><br> * paragraphs [0003], [0016], [0027], [0044], [0048], [0070] * <br> * figures 1, 2, 6, 11 * <br> ----- | 1-3,5, 8-12,15, 16,18 | |
| Y | US 2020/296528 A1 (BEEDHAM MARTYN [CH] ET AL) 17 September 2020 (2020-09-17) <br> * paragraph [0050] * <br> * figure 1 * <br> ----- | 13,14 | TECHNICAL FIELDS SEARCHED (IPC) <br><br> A61B |

The present search report has been drawn up for all claims

| Place of search | Date of completion of the search | Examiner |
|---|---|---|
| The Hague | 30 May 2023 | Worms, Georg |

EPO FORM 1503 03.82 (P04C01)

## ANNEX TO THE EUROPEAN SEARCH REPORT
## ON EUROPEAN PATENT APPLICATION NO.

EP 23 15 2070

This annex lists the patent family members relating to the patent documents cited in the above-mentioned European search report.
The members are as contained in the European Patent Office EDP file on
The European Patent Office is in no way liable for these particulars which are merely given for the purpose of information.

30-05-2023

| Patent document cited in search report | | Publication date | Patent family member(s) | | Publication date |
|---|---|---|---|---|---|
| WO 2020257188 | A1 | 24-12-2020 | NONE | | |
| US 2007112279 | A1 | 17-05-2007 | BR | PI0617377 A2 | 26-07-2011 |
| | | | CA | 2623598 A1 | 24-05-2007 |
| | | | CN | 101309629 A | 19-11-2008 |
| | | | CN | 101933808 A | 05-01-2011 |
| | | | EP | 1947999 A1 | 30-07-2008 |
| | | | US | 2007112279 A1 | 17-05-2007 |
| | | | WO | 2007058699 A1 | 24-05-2007 |
| US 2020296528 | A1 | 17-09-2020 | CN | 111557099 A | 18-08-2020 |
| | | | EP | 3718315 A1 | 07-10-2020 |
| | | | US | 2020296528 A1 | 17-09-2020 |
| | | | WO | 2019105522 A1 | 06-06-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Instruments for the measurement of aural acoustic impedance/admittance (International Electrotechnical Commission, Geneva, Switzerland),* 2004
- Measurement of eardrum acoustic impedance. **ALLEN, J. B.** Peripheral Auditory Mechanisms. Springer-Verlag, 1986, 44-51
- **NØRGAARD et al.** A coupler-based calibration method for ear-probe microphones. *J. Acoust. Soc. Am.,* 2018, vol. 144 (4), 2294-2299

- **NØRGAARD, K. R. ; FERNANDEZ-GRANDE, E. ; LAUGESEN, S.** Incorporating evanescent modes and flow losses into reference impedances in acoustic Thévenin calibration. *J. Acoust. Soc. Am.,* 2017, vol. 142 (5), 3013-3024
- *Occluded-ear simulator for the measurement of earphones coupled to the ear by means of ear inserts (International Electrotechnical Commission, Geneva, Switzerland),* 2010